# EUROPÄISCHE PATENTSCHRIFT

(11) **EP 3 189 869 B1**
(45) Veröffentlichungstag und Bekanntmachung des Hinweises auf die Patenterteilung: **03.07.2019**
(21) Anmeldenummer: 17154293.9
(22) Anmeldetag: 30.09.2010
(51) Int. Cl.: A61M 15/00

(54) **PULVERINHALATOR**
POWDER INHALER
INHALATEUR DE POUDRE

(30) Priorität: 02.10.2009 EP 09172107
(43) Veröffentlichungstag der Anmeldung: 12.07.2017
(62) Teilanmeldung aus: 10768202.3
(73) Patentinhaber: Boehringer Ingelheim International GmbH, 55216 Ingelheim am Rhein (DE)
(72) Erfinder: KAEMPER, Markus, 55216 Ingelheim am Rhein (DE); SCHULZ, Joern-Eric, 55216 Ingelheim am Rhein (DE)
(74) Vertreter: Simon, Elke Anna Maria

(56) Entgegenhaltungen:
- WO-A1-2007/118801
- WO-A1-2009/013218
- WO-A2-2008/065403
- FR-A1- 2 352 556
- US-A- 2 946 332
- US-A- 4 338 931

## Beschreibung

Die Erfindung bezieht sich auf ein Austauschrohr für einen Inhalator nach dem Oberbegriff des Anspruchs 1 bzw. auf einen Inhalator nach dem Oberbegriff des Anspruchs 9.

Ein aus dem Stand der Technik bekannter Inhalator wird beispielsweise in der EP 0 703 800 B1 oder EP 0 911 047 A1 beschrieben. Der aus den vorstehend genannten Druckschriften bekannte Inhalator weist ein schalenförmiges Unterteil und einen hierzu passenden ebenfalls schalenartigen Deckel auf, welche über ein im Randbereich angeordnetes Gelenk zur Benutzung auseinander geklappt werden können. Zwischen dem Unterteil und dem Deckel greifen in dem Gelenk noch ein ebenfalls wegklappbares Mundstück und eine darunter befindliche Platte mit darunter angeordneter Kapselhalterung an. Nach dem Auseinanderklappen der einzelnen Baugruppen kann der Patient eine mit Arzneimittel gefüllte Kapsel in die Kapselhalterung einstecken, die Platte mit Kapselhalterung sowie das Mundstück in das Unterteil zurückschwenken und die Kapsel über ein seitlich aus dem Unterteil herausragendes federvorgespanntes Betätigungsorgan anstechen. Durch ein Saugen an dem Mundstück gelangt dann das Arzneimittel in die Atemwege des zu behandelnden Patienten.

Die WO2007/118801 A1 offenbart einen Pulverinhalator, zur Verabreichung von einem Arzneimittel in Form inhalationsfähiger Substanzen, Substanzformulierungen oder - mischungen mit einem Gehäuse, das eine Kammer (4) zur Aufnahme einer das Arzneimittel aufnehmenden Kapsel aufweist. Die Kammer ist zur einmaligen Verwendung ausgebildet und enthält die Kapsel. Die Kammer ist austauschbar in dem Gehäuse angeordnet. Hierzu ist die Kammer in einer Kammeraufnahme einer schwenkbar an dem Gehäuse angelenkten sowie mit dem Gehäuse verrastbaren Platte angeordnet. Zum Öffnen der Kapsel weist die Kammer auf ihrem Umfang zwei zueinander beabstandete Bohrungen auf, in die Nadeln zum jeweils endseitigen Einstechen der Kapsel eingreifen, wobei die Nadeln in der Kammeraufnahme verschiebbar gelagert und mittels eines Betätigungselementes beaufschlagbar sind.

Nach einer Weiterbildung ist die Kammer im Querschnitt im Wesentlichen zylindrisch ausgebildet, wobei die in Richtung eines Mundstückes weisende Stirnseite mit einem zu dem Mundstück des Inhalators führenden Inhalationskanal verbunden ist und die dem Mundstück gegenüberliegende Stirnseite mit einer Lufteintrittsöffnung des Gehäuses in Wirkverbindung steht.
Der Erfindung liegt die Aufgabe zugrunde, den aus dem Stand der Technik bekannten Inhalator im Hinblick auf die Handhabung noch weiter zu verbessern.

Neben dem beschriebenen Inhalator, der einen Ausgangspunkt für die Erfindung darstellt, finden sich im Stand der Technik weitere Inhalatoren:
Die WO2008/065403 A2 offenbart ein Nasalgerät, bei dem ein Anwender in ein Mundstück ausatmet, die ausgeatmete Luft eine partikuläre Substanz mitreißt und durch ein Nasenstück in die Nase des Anwenders gebracht wird. Das Nasenstück ist hierbei austauschbar und beinhaltet einen Behälter z.B. in Form einer Kapsel oder eines Blisters mit abgemessener partikulärer Substanz. Das Gerät setzt sich aus der Nasenstückeinheit und einer Hauptbaugruppe zusammen, die ein Gehäuse, eine Mundstückeinheit und eine Substanz-Bereitstellungseinheit umfasst. Die Substanz-Bereitstellungseinheit ist fluidisch zwischen Mundstück und Nasenstück angeordnet und beinhaltet einen Reißmechanismus zum Zerreißen des Behälters. Der gezeigte Reißmechanismus umfasst einen Druckknopf einer Rückstellfeder und mit zwei Nadeln zum Anstechen eines kapselförmigen Behälters.

Die FR2352556 offenbart einen Nasalinhalator mit einer an einer Seite geschlossenen zylindrischen Wirbelkammer mit einem tangentialen Lufteinlass und einem axialen Auslass und mit einem Pulveraufnahmegehäuse, das fluidisch mit dem Lufteinlass verbunden ist. Als Pulveraufnahmegehäuse wird eine Kapsel in einer Röhre gezeigt, wobei die Kapsel der Länge nach von einer Nadel durchbohrt wird, wenn die Röhre in den Lufteinlass eingesetzt wird, so dass Luft durch die Kapsel strömen kann. In einer Variante besteht das Pulveraufnahmegehäuse aus einer zylinderförmigen Röhre mit zwei durchstechbaren Deckeln zwischen denen sich das Pulver befindet.

Die US4338931 offenbart ein Gerät zur Inhalation von pulverförmigem Arzneimittel aus einer anstechbaren Kapsel. Das Gerät umfasst zwei teleskopisch in einander verschiebbare, zylinderförmige Bauteile, die jeweils mit einer hohlen Anstechnadel ausgestattet sind. Zum Betrieb wird die Kapsel zwischen die beiden Anstechnadeln eingesetzt, die Bauteile zusammengesetzt und zusammengeschoben. Durch Zusammenschieben der Bauteile wird die Kapsel an beiden Enden angestochen. Beim Ansaugen an einem Ende kann Luft am anderen Ende des Geräts eintreten, worauf die Kapsel der Länge nach durchströmt wird und das Pulver darin sich in den Luftstrom mischt und am ersten Ende inhaliert werden kann.

Die US2946322 offenbart Gerät zum Einblasen von medizinisch wirksamem Pulver in den Nasenraum. Hierbei befindet sich das Pulver in einer Hartgelatinekapsel, die zum Gebrauch in einen Lauf eingesetzt wird, an dessen anderen Ende sich ein Gummibalg zur Drucklufterzeugung befindet. Über der Kapsel wird der Lauf dann mit einem Düsenstück verschlossen, auf das eine Kappe mit einer innenliegenden hohlen Lanze angesetzt wird, wobei die Lanze durch die Düsenöffnung hindurch die Kapsel der Länge nach durchsticht. Zum Gebrauch wird die Kappe mit der Lanze wieder heraus gezogen, und bei Betätigung des Balgs trägt die damit erzeugte Druckluft das Pulver aus der Kapsel durch die Düse in den Nasenraum.

Die Aufgabe wird erfindungsgemäß mit einem Austauschrohr für einen Inhalator gemäß Anspruch 1 und einem Inhalator mit den Merkmalen des Anspruchs 9, bzw. einer Kombination aus Austauschrohr und Inhalator gemäß Anspruch 8 gelöst.

Die Unteransprüche stellen vorteilhafte Ausgestaltungen der Erfindung dar.

Der Inhalator für die Inhalation pulverförmiger Arzneimittel aus Kapseln, beinhaltet ein Mundstück, eine in einem Unterteil eingebrachte Halterung, ein Betätigungsorgan, das aus einer Ruhestellung in eine Bewegung versetzbar ist und dabei mit mindestens einer in die Halterung einstoßbaren Nadel zusammenwirkt. In die Halterung ist ein Austauschrohr einsetzbar, das eine Kapselkammer mit der Kapsel aufweist.

Ein Merkmal der Erfindung ist, dass der Inhalator mit Austauschrohr eine Membran aufweist, mit der Pulver, das nach dem Anstich der Kapsel an den Nadeln haftet, beim Herausgleiten der Nadeln aus der Kapselkammer von den Nadeln abgestreift wird, so dass die Nadeln für die nächste Anwendung gereinigt sind. Bei der Membran kann es sich um einen Wandbereich der Kapselkammer, also des Austauschrohrs, oder um eine Membran im Bereich einer Nadeldurchführung in der Halterung handeln.

Der Inhalator für die Inhalation pulverförmiger Arzneimittel aus Kapseln, umfasst
- ein Unterteil,
- eine im Unterteil aufgenommene Platte, und eine in dem Unterteil eingebrachte Halterung,
- ein mit dem Unterteil auf der Platte verrastbares Mundstück,
- einen Deckel, der das Mundstück in einer Verschlussposition abdeckt und mittels eines Verschlusselementes verrastet, wobei das Unterteil und der Deckel durch eine Achse miteinander drehbar verbunden sind, und
- ein Betätigungsorgan, das aus einer Ruhestellung in eine Bewegung versetzbar ist und dabei mit mindestens einer in die Halterung einstoßbaren Nadel, die sich in einer Nadelhalterung des inneren Betätigungsorgans befindet, zusammenwirkt,
In die Halterung ist ein Austauschrohr als Austragungskanal einsetzbar, das eine Kapselkammer mit der Kapsel aufweist.

Aufgrund dieser Maßnahmen ist eine verbesserte Bedienbarkeit der Inhalators für die Inhalation pulverförmiger Arzneimittel aus Kapseln gegeben, da vor der Benutzung des Inhalators die Kapsel gemeinsam mit dem Austauschrohr in Form eines Wegwerfteils, das einen Austragungskanal für das Pulver bildet, direkt in die in dem Inhalator angeordnete Halterung eingesteckt wird.

Hierdurch ergibt sich für den Benutzer eine vereinfachte Handhabung, da es Patienten mit einer verminderten oder erschwerten Greiffähigkeit ihrer Hände aufgrund der Größe leichter fällt, das Austauschrohr mit der darin aufgenommenen Kapsel einzusetzen als eine Kapsel separat in eine Kapselkammer einzuführen, wie es der Stand der Technik lehrt. Außerdem werden nach der Zerstäubung bzw. Inhalation alle nennenswert mit Pulver behafteten Komponenten des Inhalators ausgetauscht, so dass bei der folgenden Benutzung ein neues Austauschrohr, also ein unbenutzter Austragskanal vorliegt.

Das neue Austauschrohr hat weiterhin einen Vorteil bei Arzneimittelformulierungen, die zu Ablagerungen im Pulveraustragsweg neigen können, da bei einem unbenutzten Austragskanal naturgemäß keine Ablagerungen durch eine vorherige Benutzung des Inhalators vorhanden ist.

Die mit jeweils mindestens einer Kapsel bestückten Austauschrohre können vom Patienten aus einer beliebigen nach heutigem Stand der Technik verfügbaren Verpackung (z B. einer Blisterverpackung, gepouchte oder ungepouchte Kartons bzw. Behälter oder einzeln gepoucht) genommen werden. Je nach den physikalischen Eigenschaften des zu inhalierenden Arzneimittels wird der Fachmann die geeignete Verpackung für das Austauschrohr mit der Kapsel wählen.

Der Inhalator entspricht in seiner Funktion, insbesondere bezüglich der Inhalation, also dem Strömungsweg des pulverförmigen Arzneimittels, dem in WO2009013218 beschriebenen Inhalator. Dies gilt insbesondere für den Anstechvorgang.

Der Inhalator besteht im Wesentlichen aus einem durch das Mundstück gebildeten Oberteil, das durch einen Deckel geschützt wird, sowie aus einem becherförmigen zweiteiligen Unterteil.

Im Innenbereich des Deckels befindet sich ein Steg, wie aus der WO2009013218 bekannt. Mit diesem Steg wird an dem erfindungsgemäßen Inhalator nicht das Mundstück sondern das Austauschrohr beim Schließen des Deckels in die Endposition gedrückt. Zudem besitzt der Deckel einen nach innen und außen gehenden Wulst, der äußerlich nicht erkennbar ist. Dieser Wulst dient dem Verschluss des Deckels am Betätigungsorgan, das sich am Unterteil des Inhalators befindet.

Um die Lösung des Deckels von dem Unterteil zu ermöglichen, besitzt das Betätigungsorgan an seiner Oberseite eine Ausnehmung, die derart geneigt ist, dass sie in Form einer schiefen Ebene eine Gleitfläche für das Verschlusselement bildet und bei Betätigung und damit Vorschieben des Betätigungsorgans den Deckel vom Unterteil löst. Diese Funktion ist nur bei vollständig eingebrachtem Austauschrohr möglich. Die Ausnehmung des Betätigungsorgans kann unterschiedlich groß sein. Die Minimalgröße muss derart sein, dass die Lösung des Deckels von dem Unterteil nach dem taschenuhrähnlichen Prinzip möglich ist. Ihre maximale Größe richtet sich nach der Oberseite des Betätigungsorgans. Die eigentliche Öffnungsbewegung des Deckels kann anschließend durch Angreifen an den Deckel durch den Patienten und vollständiges Wegklappen des Deckels vorgenommen werden. Neben dem Lösen der Verrastung durch das Betätigungsorgan kann durch geeignete Wahl der Größe der Ausnehmung am Betätigungsorgan und der Wulst am Deckel die Rückhaltekraft auch manuell durch den Patienten überwunden werden, in dem er den Deckel manuell ohne Betätigung weiterer Elemente über die Verrastung zieht.

In einer Ausführungsform kann zur Unterstützung der Öffnungsbewegung beispielsweise ein Federelement zwischen dem Deckel und/oder dem, insbesondere zweischaligen, Unterteil angeordnet sein, so dass bei einer entsprechenden Dimensionierung ein schnappartiges Aufspringen des Deckels erfolgt. Alternativ ist selbstverständlich auch eine Ausführungsform des Inhalators ohne ein Federelement zwischen dem Deckel und dem Unterteil möglich. Dabei kann das Aufschnappen auch durch geeignete Wahl des Wulstes erfolgen.

Das Betätigungsorgan ist, insbesondere bei einem beginnenden Asthmaanfall, von großer Wichtigkeit. Durch die wirksame Anordnung des Betätigungsorgans, verbunden mit einem reduzierten Kraftaufwand für den Patienten ist die Anwendung des Inhalators deutlich erleichtert. Dies gilt insbesondere, wenn Patienten an Arthritis oder ähnlichen Erkrankungen leiden oder anderweitig in der Beweglichkeit ihrer Finger eingeschränkt sind.

Das Betätigungsorgan besteht aus zwei Bauteilen derart, dass es ein Innenteil und ein Außenteil umfasst. Das Innenteil besitzt zwei parallele Führungsarme. Die Führungsarme ragen in das Unterteil hinein und dienen mit entsprechenden Einbauteilen, beispielsweise mit außenseitig an der Halterung oder innenseitig an den Unterteilhälften angeordneten Führungsstegen oder -hülsen, der Führung des Betätigungsorgans während der Bewegung aus der Ruhestellung in die jeweiligen Funktionsstellungen und zurück in die Ruhestellung. Vorzugsweise weist das Betätigungsorgan Befestigungselemente, z.B. in Form von Schnapphaken, auf, die sowohl eine sichere Montage als auch eine robuste Funktion des Gesamtgerätes gewährleisten. Das schwenkbar gelagerte Außenteil des Betätigungsorgans drückt bei einer Betätigung über eine abgerundete Anlagefläche auf das Innenteil, das sich dann linear bewegt, um die Nadeln in die Kapsel zu stechen. Die Anlagefläche kann ebenfalls sowohl auf dem Innenteil als auch auf dem Außenteil, vorzugsweise nur auf dem Innenteil, ausgebildet sein.

Das Betätigungsorgan ist mit dem Unterteil bzw. dessen beiden Unterteilhälften verbunden. Dies kann beispielsweise mittels Schnapphaken, Rasthaken oder ähnlichen technischen Lösungen geschehen. Darüber hinaus kann das Betätigungsorgan beweglich zwischen den Unterteilhälften in Freiräumen gelagert sein.

Vorzugsweise ist das Betätigungsorgan beweglich am Unterteil oder der Halterung gelagert. Eine Lagerung am Mundstück ist ebenfalls denkbar. Die Unterteilhälften bzw. das Unterteil umfassten bzw. umfasst vorzugsweise seitlich angebrachte Führungsstege, die sich aber auch an der Platte befinden und im Unterteil bzw. in den Seitenschalen des Unterteils abstützen können.

In einer günstigen Ausgestaltung ist das Betätigungsorgan federvorgespannt. Aufgrund der bereits in der Ruhestellung auftretenden Rückstellkraft ist sichergestellt, dass nach der Benutzung des Betätigungsorgans dieses in die Ruhestellung zurückgeführt wird und somit der Inhalationsvorgang begonnen bzw. fortgeführt werden kann. Die Führungsarme können an ihrem hauptkörperfernen Ende Endanschläge aufweisen, welche in der Ruhestellung an der Halterung oder den Führungshülsen anliegen. Dadurch wird eine Federspannung des Betätigungsorgans aufgebaut und reproduzierbar, die eine exakte Position aller notwendigen Bauteile in der Funktionsstellung gewährleistet. Die Führungsarme können eine beliebige Form und Anordnung haben (z.B. zusammen- oder auseinanderlaufend). Es können weiterhin ein oder mehr als zwei Führungsarme vorhanden sein. Die Ausführung kann im Querschnitt in Anlehnung an eine runde Form oder beliebig profiliert ausgestaltet sein.

Insgesamt weist das Betätigungsorgan mindestens einen Anschlagsbereich auf, der beliebig am Drückerinnen- und/oder Drückeraußenteil vorhanden sein kann. In einer bevorzugten Ausgestaltung kann der Hauptkörper des Betätigungsorgans außen mindestens eine Riffelfläche aufweisen. Diese Riffelfläche kann oben, unten oder seitlich sein. Vorzugsweise wird die Riffelfläche in mindestens einer Griffmulde des Betätigungsorgans sein. Die Riffelfläche dient sowohl als Design-Element als auch der optimalen Griffigkeit bei der Betätigung. Sie liegt am Hauptkörper des Betätigungsorgans außerhalb des Inhalationsbereiches und kommt demzufolge nicht mit dem Mundbereich des Patienten in Berührung. Darüber hinaus kann die Riffelfläche flächenmäßig kleiner als die Gesamtfläche des Betätigungsorgans ausgeführt sein und bietet dennoch eine Gewähr für eine sichere und schnelle Benutzung des Inhalators. Im Weiteren ist es selbstverständlich möglich, auch das Unterteil und/oder den Deckel mit Riffelflächen zu versehen, um die Handhabung des Inhalators weitergehend zu verbessern.

Zum besseren Verständnis der Erfindung wird diese nunmehr anhand der nachfolgenden Zeichnungen näher erläutert. Dabei zeigt:
- Fig. 1:: eine Explosionsdarstellung eines Inhalators nach dem Stand der Technik,
- Fig.2:: eine Explosionsdarstellung eines erfindungsgemäßen Inhalators und
- Fig. 3:: eine Schnittdarstellung des Inhalators nach Fig. 2.

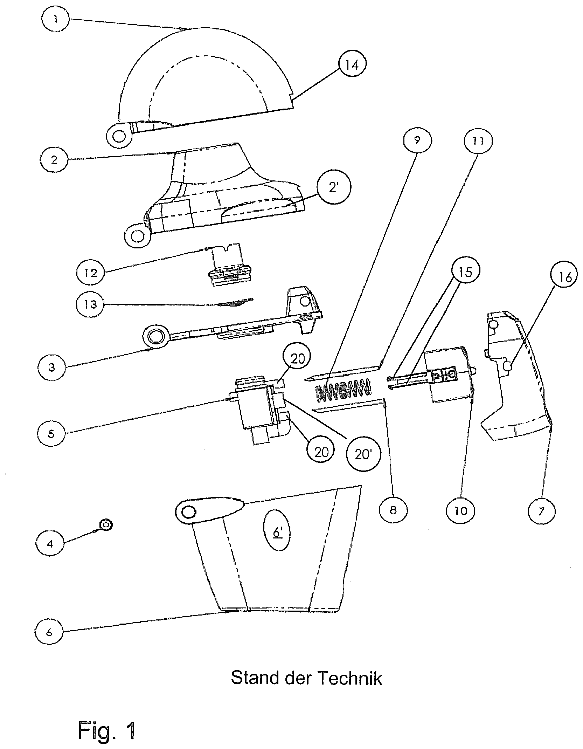

Fig. 1 zeigt einen Inhalator aus dem Stand der Technik. Die wesentlichen Baugruppen des Inhalators sind ein Unterteil 6, welches eine Platte 3 aufnimmt und durch diese abgedeckt wird, ein an der Platte 3 über Haltenasen eines Siebgehäuses 12 verrastbares Mundstück 2 und ein komplementär zu dem Unterteil 6 ausgebildeter Deckel 1. Die Platte 3, das Unterteil 6, das Mundstück 2 und der Deckel 1 sind auf einer Achse 4 gelagert.

In geschlossenem Zustand des Inhalators greift ein Verschlusselement 14 am Deckel 1 an ein äußeres Betätigungsorgan 7 und wird dort kraftschlüssig gehalten. Auch ist es möglich, durch wulstartige Ausbildungen am Verschlusselement 14 einen Formschluss zu realisieren. Für ein Angreifen des Verschlusselementes 14 am Deckel 1 weist das äußere Betätigungsorgan 7 eine Ausnehmung auf, in die das Verschlusselement 14 beim Verschließen hineinversenkt wird.

Das Betätigungsorgan besteht aus dem äußeren Betätigungsorgan 7 und einem inneren Betätigungsorgan 10. Zum Öffnen des Deckels 1 kann zunächst das äußere Betätigungsorgan 7 in Richtung des Inhalators bewegt bzw. gedrückt werden. Dabei stößt das Verschlusselement 14 am Deckel 1 auf die Ausnehmung, die bei weiterem Vorschub des Verschlusselementes 14 als Gleitfläche wirkt und für ein Lösen des Deckels 1 sorgt. Ebenso kann der Deckel 1 ohne Drücken des Betätigungsorgans 7 manuell vom Benutzer angehoben und somit der Inhalator geöffnet werden.

Die Aussparung 16 verbindet das äußere und das innere Betätigungsorgan 7, 10 mittels einer Aufhängung, die beispielsweise als Schnapphaken, Stift oder einer anderen Aufhängevorrichtung ausgebildet sein kann. Die Aussparung 16 kann von runder, ovaler oder asymmetrischer Form sein. Die Aussparung 16 kann in horizontaler oder vertikaler Position bzw. jeder Position angeordnet sein. Vorzugsweise handelt es sich bei der Aussparung 16 um ein sog. Langloch, d. h. ein längliches Oval, welches die Nadelführung in axialer Richtung optimal ermöglicht, um so ein präzises Anstechen der Kapsel zu gewährleisten.

Das Unterteil 6 ist becherartig ausgeformt und nimmt vollständig eine an der Unterseite der Platte 3 angeordnete Kapselhalterung 5 auf. Um eine mit Arzneimittel gefüllte Kapsel (nicht dargestellt) in die Kapselhalterung 5 einlegen zu können, muss jedoch auch noch das Mundstück 2 weggeklappt werden. In der Ausführungsform gemäß der Fig. 1 erfolgt dieses durch Angreifen an die am Mundstück 2 vorhandene Öffnungshilfe 2'.

In dieser geöffneten Position von Deckel 1 und Mundstück 2 kann die Kapsel durch eine Öffnung in der Platte 3 in die Kapselhalterung 5 eingelegt werden. Nachfolgend wird das Mundstück 2 wieder zurückgeschwenkt und durch Verrastung der Haltenasen des kraftschlüssig mit dem Mundstück verbundenen Siebgehäuses 12 in der Platte 3 wieder verschlossen. Das Siebgehäuse 12 beinhaltet mittig das Siebgeflecht 13. Das Siebgeflecht 13 besteht aus handelsüblichen Materialien, wie z. B. Metall oder Kunststoff. Im letzteren Fall kann das Sieb mittels des Spritzießverfahrens hergestellt werden. Zum Freisetzen des Wirkstoffes wird das äußere Betätigungsorgan 7 betätigt. Seine Ausbildung ist derart, dass sich der Nadelhalter oberhalb des Krafteinwirkungspunktes und unterhalb der Tastenaufhängung befindet. An dem inneren Betätigungsorgan 10 befindet sich mindestens eine Nadel, vorzugsweise sind es jedoch zwei, senkrecht versetzte und parallel verlaufende Nadeln 8, 11, die sich kontinuierlich mit dem Einschieben des Betätigungsorgans 7, 10 in Richtung der Kapsel (nicht dargestellt) bewegen und diese perforieren. Der Perforiervorgang kann durch ein Sichtfenster 6' beobachtet werden.

In der Kapselhalterung 5 sind zwei rohrförmige Nadeldurchführungen 20 zu erkennen, die axial entsprechend der Bewegungsrichtung der Nadeln 8, 11 ausgerichtet sind. Somit ist einerseits für ein treffsicheres Aufsetzen der Nadeln 8, 11 auf der Kapsel (nicht dargestellt) gesorgt und andererseits für eine zusätzliche Führung des Betätigungsorgans 7, 10. Die wesentliche Führung wird jedoch über zwei seitlich angeordnete Führungshülsen 20' realisiert. Führungsarme 15 haben im Zusammenspiel mit den Führungshülsen 20' die Aufgabe, das Betätigungsorgan 7, 10 unter einer Vorspannung zu halten. Hierfür sind die Führungsarme 15 an ihrem hauptkörperfernen Ende mit Endanschlägen versehen, die in der Ruhestellung des Betätigungsorgans 7, 10 an den Führungshülsen der Kapselhalterung 5 anliegen. Die Führungshülsen befinden sich an der Außenseite der Kapselhalterung 5. Zwischen den Führungsarmen 15 ist eine Schraubenfeder 9 angeordnet, die in ihrer axialen Erstreckung parallel zu den Nadeln 8, 11 verläuft, wobei die Schraubenfeder 9 derart mit der Länge der Führungsarme 15 abgestimmt ist, dass das Betätigungsorgan 7, 10 auch in der Ruhestellung noch vorgespannt ist.

Die einzelnen Baugruppen Unterteil 6, Platte 3, Mundstück 2 und Deckel 1 werden über Gelenkaufhahmen und eine Achse 4 miteinander verbunden und sind alle um diese Achse 4 herum gegenseitig verschwenkbar.

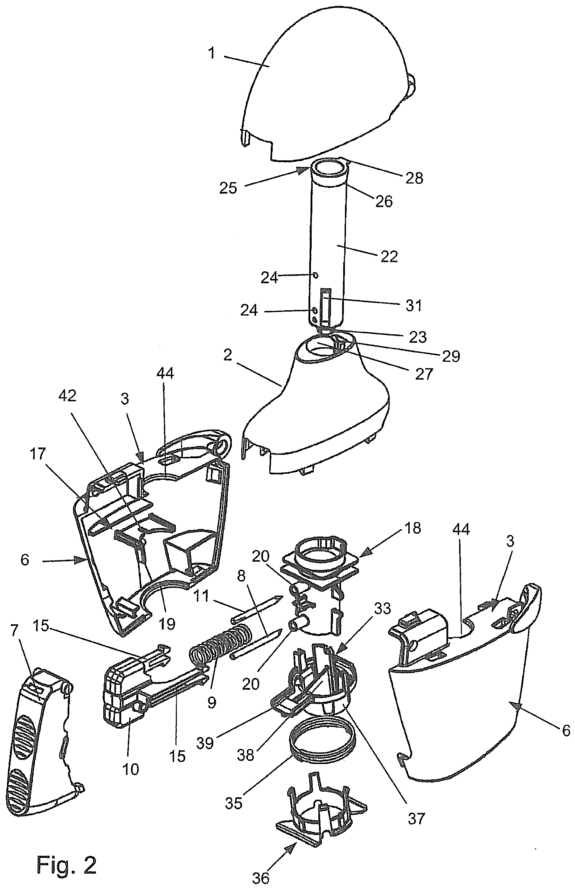

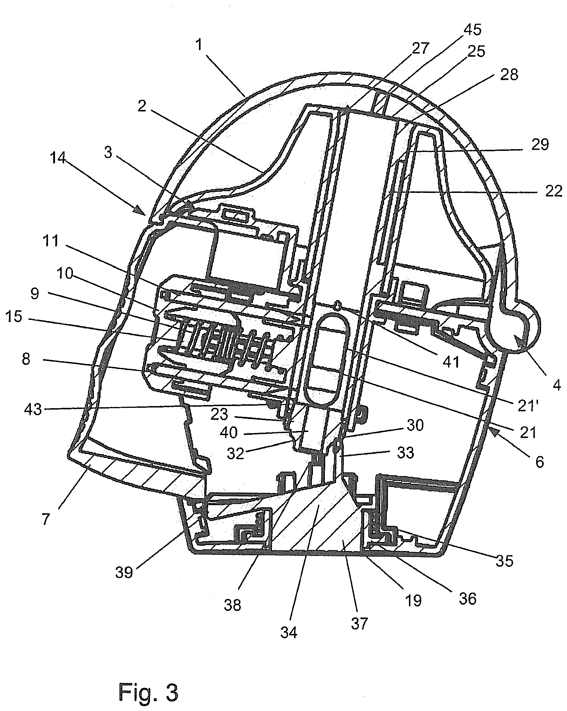

Bei dem Inhalator gemäß den Fig. 2 und 3 ist, im Gegensatz zu dem oben beschriebenen Inhalator, das Unterteil 6 zweiteilig ausgebildet und umfasst als integrales Bauteil die Platte 3. In dem Untereil 6 sind seitliche Haltestege 17, die jeweils eine Aussparung 42 aufweisen, für eine Halterung 18 und eine bodenseitige Öffnung 19 vorgesehen. eine Öffnung 44 in der Platte 3

Die Halterung 18 ist mit den beiden rohrförmigen Nadeldurchführungen 20 versehen und wird von den in den Unterteilen 6 angeordneten Haltestegen 17 mit den Aussparung 42 und der Öffnung 44 in zweiteiligen Platte 3 gehalten.

Die Kapsel 21' mit dem zu inhalierenden Arzneimittel befindet sich in einer Kapselkammer 21 in einem so genannten Austauschrohr 22, das durch das Mundstück 2 und die Halterung 18 in den Inhalator eingeschoben wird. Das Austauschrohr 22 besteht im Wesentlichen aus einer Kunststoffröhre, die von unten mit der Kapsel befüllt und mit einem, vorzugsweise ebenfalls aus einem Kunststoff bestehenden, Stopfen 23 verschlossen wird, wobei der Stopfen 23 mit einer Axialbohrung 40 zur Bildung eines Strömungskanals versehen ist. Durch einen in dem Austauschrohr 22 angespritzten Quersteg 41 ist die Kapsel nach oben hin gegen Hinausfallen gesichert und ihr für eine Vibration beim Inhalieren erforderlichen Bewegungsraum, also die Kapselkammerlänge, bestimmt. Bevorzugt ist der Stopfen 23 ein separates, nach Montage am Austauschrohr 22 nicht wieder entfernbares Bauteil. An seiner Unterseite weist der Stopfen 23 funktionale Schrägen bzw. Flächen 32 auf, die für das Auswerfen des Austauschrohres 22 benutzt werden können. Der obere Teil des Austauschrohres 22 bildet den gesamten Luftweg ab, den die zerstäubte Pulverformulierung vor Einatmung durch den Patienten im Inhalator durchläuft.

Alternativ kann der Stopfen 23 auch fest an das Austauschrohr 22 angespritzt und der Quersteg 41 zur Einbringung der Kapsel als separat montierbares Bauteil vorgesehen sein. Eine weitere Alternative kann ein komplett mit Stopfen 23 und Quersteg 41 gespritztes Austauschrohr 22 sein, das zur Einbringung der Kapsel eine verschließbare seitliche Öffnung aufweist. Im Weiteren ist es auch möglich, das Austauschrohr 22 mehrteilig zu gestalten.

Der Luftweg des Austauschrohres 22 ist an eine Inhalation der zu inhalierenden Wirkstoffe angepasst, so dass im Vergleich zu dem Inhalator aus dem Stand der Technik der Innendurchmesser des Luftwegs größer oder kleiner sein kann. Zum Erreichen der geeigneten Pulverbereitstellung bildet der Quersteg 41 sowohl die notwendige Höhe des Kapselkammer 21 innerhalb des Austauschrohres 22, damit die Kapsel vibrieren kann, als auch einen der Wirkstoffformulierung angepassten Luftwiderstand.

Das Austauschrohr 22 schließt nach dem Einschieben in den Inhalator oberseitig in seiner Gebrauchslage bündig mit dem Mundstück 2 ab, so dass der Benutzer keinen Absatz wahrnehmen kann. In dieser Position fluchten die beiden rohrförmigen Nadeldurchführungen 20 der Halterung 18 mit umfangsseitigen Öffnungen 24 für die Nadeln 8, 11 im Austauschrohr 22. Weiterhin ist es möglich, dass ein Teil des Mundstücks 2 auch im Außenbereich zu dem Austauschrohr 22 zählt, so dass der Abschluss des Austauschrohres 22 mit dem Mundstück 2 an einer beliebigen Stelle sein kann. Damit gewährleistet ist, dass das Austauschrohr 22 richtig eingesetzt wird, ist im Inhalator eine Mechanik integriert, die verhindert, dass das Betätigungsorgan 7, 10 vor der Einnahme der korrekten Endposition des Austauschrohres 22 betätigt werden kann, um mit den Nadeln 8, 11 die Kapsel einzustechen. Die Blockade des Betätigungsorgans 7, 10 kann derart erfolgen, dass überhaupt keine Betätigung möglich ist, also auch der Deckel 1 nicht geöffnet werden kann, vorzugsweise ist das Verschlusselement derart ausgelegt, dass ein Öffnen des Deckels 1 bei einem nicht eingesetzten Austauschrohr 22 möglich ist. Vorzugsweise kann die Lage des Austauschrohres 22 durch eine farbige Markierung am oberen Bereich des Austauschrohres 22 optisch erkennbar sein. Bei sachgemäßer Einbringung des Austauschrohrs 22 in die Endlage verschwindet die Markierung im Mundstück 2 und ist dann nicht sichtbar. Im Weiteren weist der das Mundstück 2 in einer Verschlussposition abdeckende Deckel 1 in seinem Inneren einen Steg 45 auf, der im geschlossenen Zustand des Deckels auf das eingebrachte Austauschrohr 22 und das Mundstück drückt.

Damit das Austauschrohr 22 lagerichtig im Inhalator positioniert wird, weist das Austauschrohr 22 im Bereich seiner dem Mundstück 2 zugeordneten Oberseite 25 einen Einführkonus 26 auf, der in der axialen Gebrauchslage des Austauschrohres 22 in einer entsprechend geformten Öffnung 27 des Mundstückes 2 einliegt. Um auch die radiale Ausrichtung des Austauschrohres 22 sicherzustellen, ist das Austauschrohr 22 im Bereich seiner dem Mundstück 2 zugeordneten Oberseite 25 mit einer radial vorstehenden Nase 28 versehen, der eine korrespondierende Nut 29 in der Öffnung 27 des Mundstückes 2 zugeordnet ist. Die Kombination von Nase 28 und Nut 29 kann bei Bedarf in entsprechenden Abmessungen oder Geometrien auch für Wirkstoffkodierungen genutzt werden, so dass ein Inhalator stets mit nur einem Wirkstoff betrieben werden kann. Zudem wurde bedacht, dass das Austauschrohr 22 unterschiedliche Außendurchmesser aufweist, so dass das Einführen zu erst mit der Oberseite 25 vermieden wird.

Im unteren Bereich des Austauschrohres 22 befindet sich eine vorliegend als umlaufende Ringnut 30, die mit dem Stopfen 23 eine Verbindung eingeht. Zudem ist ein radial wirksamer Rasthaken 43 lösbar vorgesehen, um das Austauschrohr 22 per Verrastung lösbar in dem Inhalator zu halten. Die hierfür notwendige Vertiefung 31 kann umlaufend sein, die Funktion kann statt dessen auch über einzelne Kerben, Stege oder ähnliches erfüllt werden. Weiterhin ist es möglich, mindestens eine Vertiefung zur Verrastung des Austauschrohrs 22 in der Halterung 18 und einen Rasthaken an dem Austauschrohr 22 vorzusehen.

Bei einer weiteren Ausgestaltung des Inhalators können die Öffnungen 24 für die Nadeln 8, 11 im Austauschrohr 22 mit einer Membran o.ä. verschlossen werden. Die Membran kann direkt durch den Spritzguss hergestellt oder durch Sonderverfahren mit derselben oder einer anderen Kunststoffkomponente, wie das Austauschrohr 22, realisiert werden. Die Membran streift Pulver, das nach dem Anstich der Kapsel an den Nadeln 8, 11 haftet, beim Herausgleiten der Nadeln 8, 11 aus der Kapselkammer 21 von den Nadeln 8, 11 ab, so dass die Nadeln 8, 11 für die nächste Anwendung gereinigt sind. Dieses ermöglicht eine mehrfache Verwendung von Nadeln 8, 11 zum Anstich von Kapseln mit Pulverformulierungen.

Bei der Membran kann es sich sowohl um einen Wandbereich der Kapselkammer 21, also des Austauschrohres 22, als auch um eine Membran im Bereich der Nadeldurchführung 20 handeln. Prinzipiell ist die gleiche Membran durchaus mehrfach nutzbar, da sie auch nach Nadelentfernung hinreichend dicht zur kapselfernen Seite hin ist und eine Dichtigkeit auch nach Mehrfachanstich noch gegeben ist. Das Prinzip ist auf alle gängigen Nadelformen, sowohl auf Hohl- als auch auf Vollmaterialnadeln, übertragbar. Als Membran sind sowohl gleiche als auch unterschiedliche Materialien bzw. Materialkombinationen und Geometrien, wie z.B. Dicke zur Einstellung der Flexibilität, möglich (auch Lösungen in einem Material mit Schichtdickenvariation sind denkbar), einsetzbar sind sowohl alle Arten von bei Raumtemperatur harten oder weichen Kunstoffen wie beispielsweise TPEs, Thermoplaste, Gummis aber auch Werkstoffe anderer (anorganischer) Gruppen, wie z.B. dünne Metallfolien etc.

Beim Anstich einer Kapsel mit vorgelagerter Membran wölbt sich die Membran beim Anstich zur Kapselseite hin ein, bevor sie diese tatsächlich durchsticht; die Membran kann die Kammerwand sein oder direkt mit der Kammerwand verbunden sein - sei es über herkömmlichen Spritzguss oder 2-Komponenten-Spritzgusstechniken oder über Montageschritte aus zwei oder mehr Bauteilen bestehen. Die Haftung der Membran kann beliebig ausgeführt sein: angespritzt oder umspritzt, geklipst, eingefügt, geklebt mit Zusatzmaterial oder z.B. über Selbstklebeeffekte des Membranmaterials, etc. Alternativ kann das Abstreifen des klebrigen Pulvers auch an einer passgenauen oder flexiblen Hülse, durch welche die Nadel 8, 11 durchgeführt wird, realisiert werden.

Beim Einsetzen des Austauschrohres 22 in den Inhalator verrastet es zunächst in einer eingeschobenen Zwischenlage beweglich. Beim weiteren Einschieben ist ein leicht erhöhter Widerstand zu überwinden, damit das Austauschrohr 22 seine lösbar verrastete Gebrauchslage einnimmt. Der Widerstand ist dadurch hervorgerufen, dass der an dem Austauschrohr 22 angreifende Rasthaken 43 an der Halterung 18 angeformt ist.

Formschlüssig unterhalb des Stopfens 23 befindet sich an der Schräge 32 an einer Seite oder umlaufend ein Rasthaken 33. Der Rasthaken 33 ist in einem Zwischenring 34 integriert und mit Hilfe einer Druckfeder 35 und eines Halterings 36 beweglich im Unterteil 6 befestigt. Der Zwischenring 34 weist einen koaxialen Betätigungsknopf 37 auf, der den Haltering 36 und die Öffnung 19 in dem Unterteil 6 zur Betätigung durch einen Benutzer zum Lösen der Verrastung durchragt.

Ist das Austauschrohr 22 mit Kapsel in den Inhalator eingeschoben (Fig. 3), erfolgt die Zerstäubung wie beim bisherigen Inhalator: Über das mit einem oder mehreren Fingern gleichzeitig betätigbare Betätigungsorgan 7, 10 sticht man die Kapsel durch die entsprechenden Öffnungen 24 im Austauschrohr 22 mit zwei Nadeln 8, 11 oben und unten an. Das Austauschrohr 22 ist orientiert im Inhalator eingebaut, so dass die Nadeln 8, 11 auch wirklich in die entsprechenden Öffnungen 24 im Austauschrohr 22 eintauchen können. Die Schraubenfeder 9 im Betätigungsorgan 7, 10 sorgt dafür, dass die Nadeln 8, 11 automatisch aus der Kapsel zurückfahren, sobald man das Betätigungsorgan 7, 10 freigibt. Die Inhalation erfolgt nach bewährtem Prinzip über die Vibration der Kapsel im Einatemsog des Patienten, sobald dieser am Mundstück 2 saugt.

Nach der Inhalation wird das Austauschrohr 22 mit der entleerten Kapsel in der Kapselkammer 21 durch Drücken des in diesem Ausführungsbeispiel federunterstützten Betätigungsknopf 37 im Boden des Inhalators, d.h. an der dem Mundstück 2 entgegen gesetzten Seite, gelöst. Dieser Betätigungsknopf 37 ist mit dem integrierten Rasthaken 33 formschlüssig mit dem Stopfen 23 verbunden.

Mittels der Federvorspannung des Zwischenrings 34 kann die Klemmverrastung des Austauschrohres 22 gelöst werden, wobei der Rasthaken 43 außer Eingriff mit der zugeordneten Vertiefung 31 kommt, und das Austauschrohr 22 wird nach oben aus dem Inhalator herausgedrückt. Je nach Konstruktion sind auch Lösungen denkbar, bei denen das Herauslösen des Austauschrohres 22 ohne Federunterstützung rein über Schieberkonzepte realisiert wird. Wichtig ist hierbei, dass eine Rückhaltefunktion für das Austauschrohr 22 in der Zwischenlage integriert ist, die gezielt, z.B. durch eine Taste, gelöst werden muss, bevor das Austauschrohr 22 händisch erneut über den Rasthaken 43 gezogen dem Inhalator entnommen werden kann. Federfreie Mechanismen oder Mechanismen, die das Austauschrohr 22 nur zum Teil auswerfen, sind ebenfalls möglich. Denkbar ist hierbei auch die Implementierung einer sekundären Rückhalteschwelle in der Zwischenlage, die das Austauschrohr 22 nach dem Auswerfen in einer aus dem Mundstück 2 hervorstehenden Position zurückhält, aus der man es dann händisch herausziehen kann.

Ist das Austauschrohr 22 entweder gerade ausgeworfen worden bzw. ein neues Austauschrohr 22 nicht vollständig, bis zum Endanschlag eingeschoben, entfaltet ein an den Zwischenring 34 angeformter radialer Riegel 39 innerhalb des Unterteils 6 seine Wirkung. Der Riegel 39 kommt aufgrund der Wirkung der Druckfeder 35 von innen derart mit dem äußeren Betätigungsorgan 7 in Kontakt, dass dessen Betätigbarkeit verhindert ist. Der Riegel 39 blockiert mit seinem freien Ende das äußere Betätigungsorgan 7, wenn entweder kein Austauschrohr 22 vorhanden ist oder das Austauschrohr 22 nicht die verrastete Gebrauchslage einnimmt. In diesem Zustand können die Nadeln 8, 11 nicht nach innen gefahren, also nicht durch ein unvollständig eingesetztes Austauschrohr 22 beschädigt werden.

Auf diese Weise ist sichergestellt, dass die Nadeln 8, 11 immer in die Öffnungen 24 des orientiert eingesetzten Austauschrohres 22 eintauchen und z.B. nicht bereits die Einschuböffnung des Austauschrohres 22 versperren und/oder beschädigt werden. Beim Einschieben des Austauschrohres 22 in seine verrastete Gebrauchslage wird der Zwischenring 34 über seinen nach oben zeigenden angeschrägten Steg 38 und dem Einwirken des Stopfens 23 auf den Rasthaken 33 gegen die Kraft der Druckfeder 35 nach unten geschoben, so dass der Riegel 39 aus dem Schwenkweg des äußeren Betätigungsorgans 7 verlagert wird. Gleichzeitig kommt der Betätigungsknopf 37 wieder in Auswerferbereitschaft, d.h. er nimmt eine idealerweise bündige Lage mit dem Unterteil 6 ein und ist für den Benutzer des Inhalators erreichbar.

Als Nadeln 8, 11 können alle dem Fachmann bekannten Nadeln 8, 11 und deren Kombinationen verwendet werden. Es kann sich hierbei um Voll- oder Hohlnadeln handeln. Vorzugsweise werden Vollnadeln verwendet. Insbesondere kann als obere Nadel (dem Mundstück zugewandt) eine Dreikantnadel mit einem Dreikantschliff verwendet werden. Als untere Nadel kann eine Standardnadel mit einem Standardschliff verwendet werden, wie zum Beispiel in der deutschen DIN-Norm festgelegt.
Alternativ kann die obere Nadel 11 eine Standardnadel mit einem Standardschliff und die untere Nadel 8 eine Dreikantnadel mit einem Dreikantschliff sein. Als zweite Alternative ist die Verwendung von zwei Dreikantnadeln mit einem Dreikantschliff oder zwei Standardnadeln mit einem Standardschliff möglich.

Als Kapseln können alle dem Fachmann bekannten Kapseln für Pulverinhalatoren verwendet werden (z.B.(Hart-)Gelatine-, Kunststoff-, Metallkapseln). Insbesondere kann in dem erfindungsgemäßen Inhalator eine Kunststoffkapsel zur Anwendung kommen, wie in WO 00/07572, EP 1 100 474 offenbart.

Der Inhalator kann ein Sichtfenster aufweisen. Dieses ist jedoch nicht erforderlich für seine bestimmungsgemäße Funktion.

Ebenso können alle Bauteile des Inhalators gemäß dem Fachmann bekannten Verfahren und kunststofftechnischen Möglichkeiten modifiziert werden. Mögliche Modifikationen sind beispielsweise Versteifungen oder Veränderungen der Wanddicke. Diese Möglichkeiten sind für die Funktionsweise des Inhalators jedoch nicht zwingend erforderlich.

Der Inhalator kann außerdem nach dem Fachmann bekannten Verfahren auf seiner Innen- und/oder Außenseite beschichtet werden.

Für die Inhalation kommen alle Arten von pulverförmigen Arzneimitteln in Betracht, deren Applikation auf inhalativem Wege therapeutisch sinnvoll sind.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Anti-histaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1 ,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8- {1-hydroxy-2-[2-(4-isopropyl-phenyl)-1,1dimethyl-ethylamino]-ethyl}- 4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1 - hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 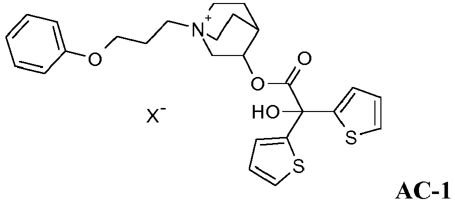 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 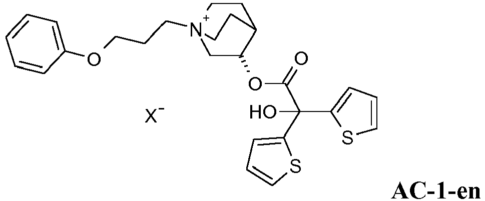 enthalten, worin X⁻ die vorstehend genannten Bedeutungen aufweisen kann. Weiterhin bevorzugte Anticholinergika sind ausgewählt aus den Salzen der Formel **AC-2** 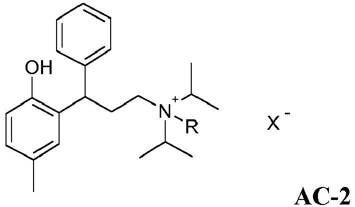 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

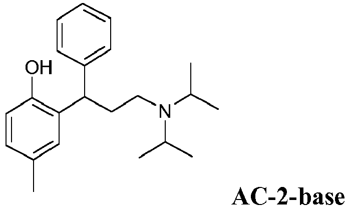

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*, 10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6- {[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl} amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1 - yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazo lin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclo hexan-1-yloxy}-7-methoxy-chinazolin
- 4-[(3-Chlor-4-fluor-phenyl)amino]-6-{trans-4-[(morpholin-4-yl)sulfonylamino]-cyclo hexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazo lin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden. Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1. | Deckel | 27. | Öffnung v. 2 |
| 2. | Mundstück | 28. | Nase |
| 2'. | Öffnungshilfe | 29. | Nut |
| 3. | Platte | 30. | Ringnut |
| 4. | Achse | 31. | Vertiefung |
| 5. | Kapselhalterung | 32. | Schräge v. 23 |
| 6. | Unterteil | 33. | Rasthaken v. 37 |
| 6'. | Sichtfenster | 34. | Zwischenring |
| 7. | äußeres Betätigungsorgan | 35. | Druckfeder |
| 8. | Nadel | 36. | Haltering |
| 9. | Schraubenfeder | 37. | Betätigungsknopf |
| 10. | inneres Betätigungsorgan | 38. | Steg v. 34 |
| 11. | Nadel | 39. | Riegel |
| 12. | Siebgehäuse | 40. | Axialbohrung |
| 13. | Siebgeflecht | 41. | Quersteg |
| 14. | Verschlusselement | 42. | Aussparung |
| 15. | Führungsarm | 43. | Rasthaken v. 23 |
| 16. | Aussparung | 44. | Öffnung v. 3 |
| 17. | Haltestege | 45. | Steg |
| 18. | Halterung | | |
| 19. | Öffnung v. 6 | | |
| 20. | Nadeldurchführung | | |
| 20'. | Führungshülse | | |
| 21. | Kapselkammer | | |
| 21'. | Kapsel | | |
| 22. | Austauschrohr | | |
| 23. | Stopfen | | |
| 24. | Öffnung v. 22 | | |
| 25. | Oberseite v. 22 | | |
| 26. | Einführkonus | | |

## Patentansprüche

1. Austauschrohr (22) zum Einsetzen als Austragungskanal in einen Inhalator für die Inhalation pulverförmiger Arzneimittel aus Kapseln, wobei der Inhalator eine Halterung (18) für das Austauschrohr (22) und ein Betätigungsorgan aufweist, das aus einer Ruhestellung in eine Bewegung versetzbar ist und dabei mit mindestens einer in die Halterung (18) einstoßbaren Nadel (8, 11), bevorzugt zwei Nadeln (8, 11), zusammenwirkt, und
wobei das Austauschrohr (22) eine Kapselkammer (21) mit einer Kapsel (21') aufweist,
**dadurch gekennzeichnet, dass**
das Austauschrohr (22) umfangsseitig Öffnungen (24) für die Nadeln (8, 11) aufweist, die durch eine Membran verschlossen sind.

2. Austauschrohr (22) nach Anspruch 1, **dadurch gekennzeichnet, dass** das insbesondere aus einem Kunststoff bestehende Austauschrohr (22) mit der Kapsel (21') zur einmaligen Inhalation verwendbar ist.

3. Austauschrohr (22) nach Anspruch 1 oder 2, **dadurch gekennzeichnet, dass** die Membran direkt mit einer Wand der Kapselkammer (21) verbunden ist.

4. Austauschrohr (22) nach Anspruch 3, **dadurch gekennzeichnet, dass** die Membran über herkömmlichen Spritzguss oder 2-Komponenten-Spritzgusstechniken direkt mit einer Wand der Kapselkammer (21) verbunden ist.

5. Austauschrohr (22) nach einem der Ansprüche 1 bis 3, **dadurch gekennzeichnet, dass** die Membran mit einer Wand der Kapselkammer (21) verbunden ist, in dem die Membran angespritzt, geklipst, eingefügt, mit Zusatzmaterial geklebt oder z.B. über Selbstklebeeffekte des Membranmaterials an der Wand der Kapselkammer (21) anhaftet.

6. Austauschrohr (22) nach einem der vorangegangenen Ansprüche, **dadurch gekennzeichnet, dass** das Austauschrohr (22) an dem die Kapselkammer (21) ausbildenden Ende mit einem Stopfen (23) verschlossen ist, wobei der Stopfen (23) mit einer Axialbohrung (40) zur Bildung eines Strömungskanals versehen ist.

7. Austauschrohr (22) nach Anspruch 6, **dadurch gekennzeichnet, dass** die Kapselkammer (21) zum einen durch den Stopfen (23) und zum anderen durch einen in das Austauschrohr (22) ragenden Quersteg (41) begrenzt ist.

8. Inhalator für die Inhalation pulverförmiger Arzneimittel aus Kapseln, umfassend
- ein Unterteil (6)
- eine in dem Unterteil (6) eingebrachte Halterung (18),
- ein Mundstück (2), und
- ein Betätigungsorgan, das aus einer Ruhestellung in eine Bewegung versetzbar ist und dabei mit mindestens einer in die Halterung (18) einstoßbaren Nadel (8, 11) zusammenwirkt,
**dadurch gekennzeichnet, dass** in die Halterung (18) ein Austauschrohr (22) gemäß einem der vorangegangenen Ansprüche eingesetzt ist.

9. Inhalator für die Inhalation pulverförmiger Arzneimittel aus Kapseln, umfassend
- ein Unterteil (6)
- eine in dem Unterteil (6) eingebrachte Halterung (18),
- ein Mundstück (2), und
- ein Betätigungsorgan, das aus einer Ruhestellung in eine Bewegung versetzbar ist und dabei mit mindestens einer in die Halterung (18) einstoßbaren Nadel (8, 11) zusammenwirkt,
**dadurch gekennzeichnet, dass** in die Halterung (18) ein Austauschrohr (22) als Austragungskanal einsetzbar ist, das eine Kapselkammer (21) mit der Kapsel (21') und umfangsseitig Öffnungen (24) für die Nadeln (8, 11) aufweist, und
dass der Inhalator eine Membran aufweist, mit der Pulver, das nach dem Anstich der Kapsel (21') an der mindestens einen Nadel (8, 11) haftet, beim Herausgleiten der mindestens einen Nadel (8, 11) aus der Kapselkammer (21) von der Nadel (8, 11) abgestreift wird,
wobei die Membran im Bereich einer Nadeldurchführung (20) in der Halterung (18) angeordnet ist

10. Inhalator nach einem der Ansprüche 8 oder 9, **dadurch gekennzeichnet, dass** das Austauschrohr (22) in seiner Gebrauchslage in dem Inhalator lösbar arretiert ist, insbesondere wobei der Inhalator einen federunterstützten Betätigungsknopf (37) mit einem integrierten Rasthaken aufweist, durch dessen Betätigung das Austauschrohr (22) aktiv gelöst werden kann.

11. Inhalator nach Anspruch 10, **dadurch gekennzeichnet, dass** an einem federnd gelagerten Zwischenring (34) ein radialer Riegel (39) angeformt ist, der derart mit dem Betätigungsorgan, zusammenwirkt, dass es bei einem nicht vorhandenen bzw. nicht in der axialen Gebrauchslage arretierten Austauschrohr (22) das Betätigungsorgan derart arretiert, dass seine Verlagerung zum Anstechen der Kapsel (21') verhindert ist, und bei einem in der axialen Gebrauchslage arretierten Austauschrohr (22) das Betätigungsorgan zum Anstechen der Kapsel (21') mit den Nadeln freigibt.

12. Inhalator nach einem der Ansprüche 8 bis 11, **dadurch gekennzeichnet, dass** das Austauschrohr (22) sich in seiner Gebrauchslage durch das Mundstück (2) erstreckt.

13. Inhalator nach einem der Ansprüche 8 bis 12, **dadurch gekennzeichnet, dass** der Inhalator eine Öffnung (27) im Mundstück (2) aufweist, wodurch das Austauschrohr (22) durch das Mundstück (2) und in die Halterung (18) in den Inhalator eingeschoben werden kann, und dass das Austauschrohr (22) im Bereich seiner dem Mundstück (2) zugeordneten Oberseite (25) eine radial vorstehende Nase (28) aufweist, der eine korrespondierende Nut (29) in der Öffnung (27) am Mundstück (2) zugeordnet ist.

## Claims

1. Exchangeable tube (22) for insertion as an expulsion channel into an inhaler for inhaling powdered medicaments from capsules, wherein the inhaler comprises a holder (18) for the exchangeable tube (22) and an actuating member that can be moved from a resting position and set in motion and at the same time co-operates with at least one pin (8, 11), preferably two pins (8,11), that can be stuck into the holder (18),
wherein the exchangeable tube (22) comprises a capsule chamber (21) with a capsule (21'),
**characterised in that,**
the exchangeable tube (22) comprises, on its circumferential side, openings (24) for the pins (8, 11), the openings (24) being closed off by a membrane.

2. Exchangeable tube (22) according to claim 1, **characterised in that** the exchangeable tube (22) with the capsule may be used for a single inhalation, the exchangeable tube (22) in particular consisting of plastic.

3. Exchangeable tube (22) according to claim 1 or 2, **characterised in that** the membrane is directly connected to a wall of the capsule chamber (21).

4. Exchangeable tube (22) according to claim 3, **characterised in that** the membrane is directly connected to a wall of the capsule chamber (21) by conventional injection moulding or by 2-component injection moulding techniques.

5. Exchangeable tube (22) according to one of claims 1 to 3, **characterised in that** the membrane is connected to a wall of the capsule chamber (21), wherein the membrane is moulded on, clipped on, inserted, glued with additional material or sticks to the wall of the capsule chamber (21) for example by the self-sticking effects of the membrane material.

6. Exchangeable tube (22) according to one of the preceding claims, **characterised in that** the exchangeable tube (22) is closed off with a stopper (23) at its end that forms the capsule chamber (21), wherein the stopper (23) is provided with an axial bore (40) to form a flow channel.

7. Exchangeable tube (22) according to claim 6, **characterised in that** the capsule chamber (21) is bounded on the one hand by the stopper (23) and on the other hand by a transverse member (41) projecting into the exchangeable tube (22).

8. Inhaler for inhaling powdered medicaments from capsules, comprising
- a lower part (6),
- a holder (18) inserted in the lower part (6),
- a mouthpiece (2), and
- an actuating member that can be moved from a resting position and set in motion and at the same time co-operates with at least one pin (8, 11) that can be stuck into the holder (18),
**characterised in that**
an exchangeable tube (22) according to one of the preceding claims is inserted in the holder (18).

9. Inhaler for inhaling powdered medicaments from capsules, comprising
- a lower part (6),
- a holder (18) inserted in the lower part (6),
- a mouthpiece (2), and
- an actuating member that can be moved from a resting position and set in motion and at the same time co-operates with at least one pin (8, 11) that can be stuck into the holder (18),
**characterised in that**
as an expulsion channel, an exchangeable tube (22) is inserted in the holder (18), the exchangeable tube (22) comprising a capsule chamber (21) with the capsule (21') and, on its circumferential side, openings (24) for the pins (8, 11), and
that the inhaler comprises a membrane with which powder adhering to the at least one pin (8, 11) after the piercing of the capsule is wiped off the pin (8,11), as the pin (8, 11) slides out of the capsule chamber (21),
wherein the membrane is arranged in the region of a pin guide (20) in the holder (18).

10. Inhaler according to one of claims 8 or 9, **characterised in that** the exchangeable tube (22) is releasably secured in its position of use in the inhaler, in particular wherein the inhaler comprises a spring-assisted actuating button (37) with an integrated latching hook (33), wherein the exchangeable tube (22) can be actively released by the operation of said button.

11. Inhaler according to claim 10, **characterised in that** a radial bar (39) is formed on a resiliently mounted intermediate ring (34), said bar cooperating with the actuating member such that, when no exchangeable tube (22) is present, or no exchangeable tube (22) is secured in the axial position of use, it secures the actuating member so as to prevent its moving to pierce the capsule (21'), and when an exchangeable tube (22) is secured in the axial position of use, the bar (39) releases the actuating member to pierce the capsule (21') with the pins.

12. Inhaler according to one of claims 8 to 11, **characterised in that**, in its position of use, the exchangeable tube (22) extends through the mouthpiece (2).

13. Inhaler according to one of claims 8 to 12, **characterised in that** the inhaler comprises an opening (27) in the mouthpiece (2) through which the exchangeable tube (22) can be inserted through the mouthpiece (2) and into the holder (18) in the inhaler, and that the exchangeable tube (22) comprises, in the region of its upper side (25) associated with the mouthpiece (2), a radially projecting lug (28) which is associated with a corresponding groove (29) in the mouthpiece (2).

## Revendications

1. Tube de remplacement (22) pour l'insertion en tant que canal de refoulement dans un inhalateur pour l'inhalation de médicaments sous forme pulvérulente issus de gélules, dans lequel l'inhalateur présente un support (18) pour le tube de remplacement (22) et un organe d'actionnement qui peut être déplacé depuis une position de repos en un déplacement et qui, ce faisant, coopère avec au moins une aiguille (8, 11) pouvant être enfoncée dans le support (18), de préférence deux aiguilles (8, 11), et
dans lequel le tube de remplacement (22) présente un compartiment à gélules (21) avec une gélule (21'),
**caractérisé en ce que**
le tube de remplacement (22) présente, côté périphérie, des ouvertures (24) pour les aiguilles (8, 11), qui sont fermées par une membrane.

2. Tube de remplacement (22) selon la revendication 1, **caractérisé en ce que** le tube de remplacement (22), composé en particulier d'une matière plastique, peut être utilisé avec la gélule (21') aux fins de l'inhalation unique.

3. Tube de remplacement (22) selon la revendication 1 ou 2, **caractérisé en ce que** la membrane est raccordée directement à une paroi du compartiment à gélules (21).

4. Tube de remplacement (22) selon la revendication 3, **caractérisé en ce que** la membrane est raccordée par le biais de techniques de moulage par injection traditionnel ou à deux composants, directement à une paroi du compartiment à gélules (21).

5. Tube de remplacement (22) selon l'une quelconque des revendications 1 à 3, **caractérisé en ce que** la membrane est raccordée à une paroi du compartiment à gélules (21), **en ce que** la membrane est injectée, clipsée, insérée, collée avec un matériau supplémentaire ou par exemple adhère à la paroi du compartiment à gélules (21) par le biais des effets autocollants du matériau de membrane.

6. Tube de remplacement (22) selon l'une quelconque des revendications précédentes, **caractérisé en ce que** le tube de remplacement (22) est fermé à l'extrémité réalisant le compartiment à gélules (21) avec un obturateur (23), l'obturateur (23) étant pourvu d'un perçage axial (40) pour la formation d'un canal d'écoulement.

7. Tube de remplacement (22) selon la revendication 6, **caractérisé en ce que** le compartiment à gélules (21) est délimité d'une part par l'obturateur (23) et d'autre part par une traverse (41) pénétrant dans le tube de remplacement (22).

8. Inhalateur pour l'inhalation de médicaments sous forme pulvérulente issus de gélules comprenant
- une partie inférieure (6),
- un support (18) introduit dans la partie inférieure (6),
- un embout buccal (2), et
- un organe d'actionnement qui peut être déplacé depuis une position de repos en un déplacement et qui, ce faisant, coopère avec au moins une aiguille (8, 11) pouvant être enfoncée dans le support (18),
**caractérisé en ce qu'**un tube de remplacement (22) selon l'une quelconque des revendications précédentes est inséré dans le support (18).

9. Inhalateur pour l'inhalation de médicaments sous forme pulvérulente issus de gélules comprenant
- une partie inférieure (6),
- un support (18) introduit dans la partie inférieure (6),
- un embout buccal (2), et
- un organe d'actionnement qui peut être déplacé depuis une position de repos en un déplacement et qui, ce faisant, coopère avec au moins une aiguille (8, 11) pouvant être enfoncée dans le support (18),
**caractérisé en ce qu'**un tube de remplacement (22) peut être inséré en tant que canal de refoulement dans le support (18) **en ce qu'**il présente un compartiment à gélules (21) avec la gélule (21') et, côté périphérie, des ouvertures (24) pour les aiguilles (8, 11), et
**en ce que** l'inhalateur présente une membrane avec laquelle de la poudre qui adhère, après le piquage de la gélule (21'), à l'au moins une aiguille (8, 11), est enlevée de l'aiguille (8, 11) lors du glissement de l'au moins une aiguille (8, 11) hors du compartiment à gélules (21),
la membrane étant agencée dans la zone d'un passage d'aiguille (20) dans le support (18).

10. Inhalateur selon l'une quelconque des revendications 8 ou 9, **caractérisé en ce que** le tube de remplacement (22) est bloqué de manière amovible dans sa position d'utilisation dans l'inhalateur, en particulier dans lequel l'inhalateur présente un bouton d'actionnement (37) assisté par ressort avec un crochet d'encliquetage intégré, par l'actionnement duquel le tube de remplacement (22) peut être détaché de façon active.

11. Inhalateur selon la revendication 10, **caractérisé en ce qu'**un verrou (39) radial est formé au niveau d'une bague intermédiaire (34) montée sur ressorts, lequel verrou coopère avec l'organe d'actionnement de telle manière qu'il bloque l'organe d'actionnement dans le cas de l'absence d'un tube de remplacement (22) ou d'un non-blocage de ce dernier dans la position d'utilisation axiale de telle manière que son déplacement servant à piquer la gélule (21') est empêché et libère, dans le cas d'un blocage du tube de remplacement (22) dans la position d'utilisation axiale, l'organe d'actionnement servant à piquer la gélule (21') à l'aide des aiguilles.

12. Inhalateur selon l'une quelconque des revendications 8 à 11, **caractérisé en ce que** le tube de remplacement (22) s'étend dans sa position d'utilisation au travers de l'embout buccal (2).

13. Inhalateur selon l'une quelconque des revendications 8 à 12, **caractérisé en ce que** l'inhalateur présente une ouverture (27) dans l'embout buccal (2), moyennant quoi le tube de remplacement (22) peut être enfoncé dans l'embout buccal (2) et dans le support (18) dans l'inhalateur, et **en ce que** le tube de remplacement (22) présente, dans la zone de son côté supérieur (25) associé à l'embout buccal (2), un nez (28) dépassant radialement auquel est associée une rainure (29) correspondante dans l'ouverture (27) au niveau de l'embout buccal (2).
